# EUROPÄISCHE PATENTANMELDUNG

(11) **EP 3 798 973 A1**
(43) Veröffentlichungstag der Anmeldung: **31.03.2021**
(21) Anmeldenummer: 20195667.9
(22) Anmeldetag: 11.09.2020
(51) Int. Cl.: G06T 7/00, G06T 19/00, A61B 6/00, A61B 6/02

(54) **VERFAHREN ZUR ERMITTLUNG ORTHOGONALER SCHICHTBILDDATENSÄTZE EINER TOMOSYNTHESEAUFNAHME**

(30) Priorität: 30.09.2019 EP 19200379
(71) Anmelder: Siemens Healthcare GmbH, 91052 Erlangen (DE)
(72) Erfinder: Radicke, Marcus, 90587 Veitsbronn (DE); Veitenhansl, Stefan, 90427 Nürnberg (DE)

(57) **Zusammenfassung**

Die Erfindung betrifft ein Verfahren (100) zur Ermittlung orthogonaler Schichtbilddatensätze einer Tomosyntheseaufnahme eines Untersuchungsbereichs, insbesondere einer Brust, aufweisend die Schritte:
- Aufnehmen (101) einer Tomosyntheseaufnahme und Rekonstruieren (102) von einer Mehrzahl von ersten Schichtbilddatensätzen in einer ersten Ebene basierend auf der Tomosyntheseaufnahme,
- Auswählen (103) eines ersten Schichtbilddatensatzes aus der Mehrzahl von ersten Schichtbilddatensätzen,
- Markieren (104) einer Mikrokalzifikation (202) oder einer interessierenden Region mit einer punktförmigen Markierung im ausgewählten ersten Schichtbilddatensatz,
- Ermitteln (105) von einem zweiten Schichtbilddatensatz in einer zur ersten Ebene orthogonalen zweiten Ebene und von einem dritten Schichtbilddatensatz in einer zur ersten Ebene und zweiten Ebene orthogonalen dritten Ebene, wobei der Schnittpunkt der ersten Ebene, der zweiten Ebene und der dritten Ebene die punktförmige Markierung umfasst.

## Beschreibung

Die Erfindung betrifft ein Verfahren zur Ermittlung orthogonaler Schichtbilddatensätze einer Tomosyntheseaufnahme eines Untersuchungsbereichs, ein Mammographiesystem, ein Computerprogrammprodukt und ein computerlesbares Medium dazu.

In der Brustbildgebung, insbesondere im Rahmen von Screening und Diagnostik, setzt sich die Brusttomosynthese mehr und mehr durch und etabliert sich möglicherweise als Standard. Dieser Übergang von einer 2D-Modalität, z.B. der digitalen Vollfeldmammographie, auf eine 3D-Modalität, der Brusttomosynthese, kann im klinischen Alltag einige Herausforderungen mit sich bringen. Im Rahmen der Erfindung wird dabei die Herausforderung des zeiteffektiven Readings der Daten und die Optimierung des Biopsieablaufes betrachtet. Die Erfinder haben dabei erkannt, dass es wünschenswert wäre, wenn der Radiologe nicht die gesamten rekonstruierten Tomosyntheseschichten befunden müsste, sondern anhand eines einzelnen Bildes eine Diagnose treffen bzw. einen suspekten Biopsiebereich identifizieren könnte.

Beispielsweise ist ein synthetisches Mammogramm bekannt, bei welchem aus den dreidimensionalen Daten ein zweidimensionales Bild berechnet wird. Der Nachteil dieses synthetischen Mammogramms ist, dass es für den Radiologen teilweise nicht ersichtlich ist, ob es sich bei einer im zweidimensionalen Bild sichtbaren lokalen Mikrokalkhäufung tatsächlich um ein lokales Cluster handelt oder ob die Mikrokalks über die im zweidimensionalen Bild nicht darstellbare Brusttiefe verteilt sind und damit kein erhöhter Malignitätsverdacht besteht.

Eine weitere Herausforderung bezüglich der Darstellung tritt im Biopsieworkflow auf. Auch hier können Kalkcluster in ihrer räumlichen Ausdehnung nicht auf einen Blick erkannt werden.

Bei einer Tomo-Biopsie muss der Radiologe durch mehrere Schichten scrollen und das Zentrum des Kalkclusters schätzen. Bei der Stereobiopsie hat man zwar zwei Ansichten, jedoch sind die Winkel effektiv zu klein, um eine vollständige räumliche Ausdehnung sicher erfassen zu können. Bisher kann die gewünschte Genauigkeit bei der Bestimmung des Zentrums des Clusters noch nicht erreicht werden. Beispielsweise ist es bisher üblich, durch dynamische Betrachtung mehrerer paralleler oder zueinander verkippter Schichten eine Schätzung der Tiefenverteilung der Mikrokalzifikationen zu erhalten. Insbesondere beim Setzen bzw. Markieren des Targets für die Biopsie wäre eine weitere visuelle Hilfestelle nützlich und wünschenswert. Des Weiteren fehlt die visuelle Information, ob die Größe der Notch zur entsprechenden Ausdehnung des Kalkclusters ausreichend ist.

Es ist Aufgabe der Erfindung, ein Verfahren zur Ermittlung orthogonaler Schichtbilddatensätze einer Tomosyntheseaufnahme eines Untersuchungsbereichs, ein Mammographiesystem, ein Computerprogrammprodukt und ein computerlesbares Medium anzugeben, welche eine verbesserte Lokalisation einer Mikrokalzifikation oder eines Clusters ermöglichen.

Die Aufgabe wird erfindungsgemäß gelöst durch ein Verfahren zur Ermittlung orthogonaler Schichtbilddatensätze einer Tomosyntheseaufnahme eines Untersuchungsbereichs nach Anspruch 1, ein Mammographiesystem nach Anspruch 13, ein Computerprogrammprodukt nach Anspruch 14 und ein computerlesbares Medium nach Anspruch 15.

Die Erfindung betrifft ein Verfahren zur Ermittlung orthogonaler Schichtbilddatensätze einer Tomosyntheseaufnahme eines Untersuchungsbereichs, insbesondere einer Brust, aufweisend die Schritte des Aufnehmens, des Rekonstruierens, des Auswählens, des Markierens und des Ermittelns. Im Schritt des Aufnehmens wird eine Tomosyntheseaufnahme aufgenommen. Im Schritt des Rekonstruierens wird eine Mehrzahl von ersten Schichtbilddatensätzen in einer ersten Ebene basierend auf der Tomosyntheseaufnahme rekonstruiert. Im Schritt des Auswählens wird ein erster Schichtbilddatensatz aus der Mehrzahl von ersten Schichtbilddatensätzen ausgewählt. Im Schritt des Markierens wird eine Mikrokalzifikation oder eine interessierende Region mit einer punktförmigen Markierung im ausgewählten ersten Schichtbilddatensatz markiert. Im Schritt des Ermittelns wird ein zweiter Schichtbilddatensatz in einer zur ersten Ebene orthogonalen zweiten Ebene und ein dritter Schichtbilddatensatz in einer zur ersten Ebene und zweiten Ebene orthogonalen dritten Ebene ermittelt, wobei der Schnittpunkt der ersten Ebene, der zweiten Ebene und der dritten Ebene die punktförmige Markierung umfasst.

Bei der Tomosyntheseaufnahme werden im Schritt des Aufnehmens üblicherweise mehrere Projektionen unter unterschiedlichen Winkeln in einem vorbestimmten Winkelbereich von einer Brust aufgenommen. Aus den Projektionen kann mit Hilfe einer Tomosyntheserekonstruktion im Schritt der Rekonstruktion eine Mehrzahl von ersten Schichtbilddatensätzen in einer ersten Ebene rekonstruiert werden. Damit kann ein Tomosynthesevolumen aus der Tomosyntheseaufnahme rekonstruiert werden. Die erste Ebene kann beispielsweise eine xy-Ebene sein. In der Mehrzahl von ersten Schichtbilddatensätzen können sowohl das Drüsengewebe bzw. das Weichteilgewebe sowie - falls vorhanden - stärker schwächende Mikrokalzifikationen dargestellt werden. Eine Mikrokalzifikation kann auch abgekürzt als sogenannter Mikrokalk bezeichnet werden.

Es kann eine Mikrokalzifikation von einem Benutzer oder mit Hilfe eines Bilderkennungsalgorithmus erkannt werden. Im Schritt des Auswählens kann beispielsweise durch den Benutzer ein erster Schichtbilddatensatz ausgewählt werden. Alternativ kann der erste Schichtbilddatensatz von einem Algorithmus, beispielsweise zur Bild- oder Mustererkennung, ausgewählt werden. Der erste Schichtbilddatensatz wird insbesondere dann ausgewählt, wenn eine Mikrokalzifikation im ersten Schichtbilddatensatz erkannt wurde. Die interessierende Region kann eine Läsion ohne Mikrokalzifikationen sein, welche mittels einer Biopsie untersucht werden soll.

Die Mikrokalzifikation kann insbesondere im Wesentlichen punktförmig im Schritt des Markierens markiert werden. Die Markierung kann beispielsweise mittels eines Klicks oder einer Touchgeste oder Berührungsgeste auf der Benutzeroberfläche, welche den ersten Schichtbilddatensatz anzeigt, markiert werden. Die Mikrokalzifikation kann automatisch markiert werden, sobald sie beispielsweise von einem Algorithmus erkannt wird. Die Anzeige kann beispielsweise den ersten Schichtbilddatensatz mit der markierten Mikrokalzifikation anzeigen. Es werden nun ein zweiter und ein dritter Schichtbilddatensatz erzeugt. Der ausgewählte erste Schichtbilddatensatz sowie der zweite und der dritte Schichtbilddatensatz können als orthogonale Projektionen basierend auf den Tomosynthesedaten bezeichnet werden.

Es wird vorgeschlagen, in der Brustbefundung und im Biopsieworkflow eine Darstellung mehrerer Ansichten mit einer quasistatischen Darstellung bereitzustellen. In den drei Views bzw. Ansichten bzw. dem ersten, zweiten und dritten Schichtbilddatensatz können jeweils eine orthogonale Projektion aus den Tomosynthesedaten dargestellt werden. Die Hauptsicht kann insbesondere den ersten Schichtbilddatensatz des Tomosynthesedatensatzes aufweisen, beispielsweise in der xy-Ebene. Die beiden anderen Ansichten bzw. der zweite und dritte Schichtbilddatensatz können als orthogonale Projektionen ausgehend von der Markierung bezeichnet werden. Die Markierung im (ausgewählten) ersten Schichtbilddatensatz umfasst den Schnittpunkt einer horizontalen Schicht, beispielsweise in der xz-Ebene, und einer vertikalen Schicht, beispielsweise in der yz-Ebene. Die horizontale Schicht bzw. der zweite Schichtbilddatensatz kann in der xz-Ebene ausgebildet sein. Die vertikale Schicht bzw. der dritte Schichtbilddatensatz kann in der yz-Ebene ausgebildet sein. Die xy-, xz- und yz-Ebene können orthogonale Ebenen des rekonstruierten Tomosynthesevolumens basierend auf der Tomosyntheseaufnahme sein. Der ausgewählte erste Schichtbilddatensatz kann insbesondere ein Schichtbilddatensatz aus dem Stapel mit der Mehrzahl von ersten Schichtbilddatensätzen bzw. dem rekonstruierten Volumen der rekonstruierten Tomosyntheseaufnahme sein. Im zweiten Schichtbilddatensatz können insbesondere die Bildwerte eines konstanten y-Wertes entsprechend der y-Koordinate der punktförmigen Markierung in Abhängigkeit von x und der Schicht z aus der Mehrzahl von ersten Schichtbilddatensätzen eingetragen werden. Im dritten Schichtbilddatensatz können insbesondere die Bildwerte eines konstanten x-Wertes entsprechend der x-Koordinate der punktförmigen Markierung in Abhängigkeit von y und der Schicht z aus der Mehrzahl von ersten Schichtbilddatensätzen eingetragen werden.

Es kann alternativ auch eine andere Orientierung des zweiten und des dritten Schichtbilddatensatzes gewählt werden. Beispielsweise können der zweite und der dritte Schichtbilddatensatz jeweils um einen Winkel, beispielsweise 45 Grad, gedreht ausgerichtet sein. Dies kann insbesondere hinsichtlich eines Biopsiepfades bzw. einer Trajektorie für die Biopsie entlang des Winkels vorteilhaft sein.

Hierdurch erhält der Radiologe oder der Benutzer einen direkten Überblick, ob die Mikrokalkansammlung bzw. der Cluster im ausgewählten ersten Schichtbilddatensatz lokal zusammenhängend ist. Vorteilhaft kann die Befundung mit nur einem Blick auf die gleichzeitig dargestellten drei Ansichten, der ausgewählte erste Schichtbilddatensatz sowie der zweite und dritte Schichtbilddatensatz, übersichtlicher und vereinfacht werden. Vorteilhaft kann eine Darstellung der orthogonalen Ebenen des 3D-Volumens basierend auf der Tomosyntheseaufnahme bereitgestellt werden.

In einem weiteren optionalen Schritt des erneuten Markierens kann, insbesondere der Benutzer, auf eine bestimmte bzw. erkannte weitere Mikrokalzifikation im ersten, zweiten oder dritten Schichtbilddatensatz klicken bzw. diese Mikrokalzifikation auswählen. In einem Schritt des Hervorhebens kann die markierte weitere Mikrokalzifikation im ersten, zweiten und dritten Schichtbilddatensatz hervorgehoben werden, beispielsweise mittels einer kreisförmigen Umrandung. Vorteilhaft kann die Lage der weiteren Mikrokalzifikation in der Nachbarschaft der markierten Mikrokalzifikation genauer lokalisiert werden.

Vorteilhaft kann der Workflow zur Diagnose gegenüber einem Workflow basierend auf FFDM-Aufnahmen beschleunigt werden. Vorteilhaft kann eine intuitivere Darstellung mit verbesserten Möglichkeiten zur genaueren Lokalisation von Mikrokalzifikationen bereitgestellt werden. Die Lage von benachbarten Mikrokalzifikationen kann vorteilhaft genauer bestimmt werden. Vorteilhaft kann eine verbesserte Diagnosequalität ermöglicht werden. Vorteilhaft kann eine Probennahme in der Biopsie genauer und vollständiger durchgeführt werden.

Gemäß einem Aspekt der Erfindung wird mindestens eine Mikrokalzifikation automatisch detektiert. Es kann vorteilhaft ein bekannter Algorithmus verwendet werden, welcher Mikrokalzifikationen in den Tomosyntheseschichten, d.h. den ersten Schichtbilddatensätzen bzw. stereotaktischen Bilder detektieren bzw. erkennen kann. Vorteilhaft kann eine Liste mit erkannten Mikrokalzifikationen erstellt werden. Vorteilhaft können, insbesondere dem Benutzer, die erkannten Mikrokalzifikationen in einer Reihenfolge angezeigt werden oder, beispielsweise in der Liste, zur Auswahl stehen. Bereiche mit mindestens einer detektierten Mikrokalzifikation können als suspekter Bereich klassifiziert werden. Es können der erste, zweite und dritte Schichtbilddatensatz für verschiedene detektierte Mikrokalzifikationen nacheinander dargestellt werden, beispielsweise kann der Benutzer zur Darstellung mit der nächsten detektierten und markierten Mikrokalzifikation springen bzw. die Darstellungen können automatisch nacheinander angezeigt werden.

Gemäß einem Aspekt der Erfindung wird bei einer Mehrzahl detektierter Mikrokalizifikationen eine automatische Clusteranalyse durchgeführt. Die automatische Clusteranalyse kann eine Weiterbildung des Algorithmus zur Erkennung von Mikrokalzifikationen sein. Alternativ können die Mikrokalzifikationen manuell vom Benutzer detektiert werden. Die Verteilung der erkannten Mikrokalzifikationen kann automatisch ausgewertet werden. Bei der automatischen Auswertung können die detektierten Mikrokalzifikationen derart ausgewertet werden, ob mehrere Mikrokalzifikationen eine Mikrokalkansammlung oder ein Cluster bilden. Dazu kann der Abstand zwischen mindestens zwei detektierten Mikrokalzifikationen ausgewertet werden. Unterschreitet der Abstand einen vorbestimmten Schwellwert, so werden die mindestens zwei detektieren Mikrokalzifikationen einem Cluster zugeordnet. Im ausgewählten ersten, zweiten und dritten Schichtbilddatensatz kann eine virtuelle Hülle bzw. Umrandung an den Clustergrenzen um die zugeordneten Mikrokalzifikationen überlagert werden. Vorteilhaft können Cluster besonders schnell und einfach in den drei Ansichten lokalisiert werden. Vorteilhaft kann eine Diagnose und auch eine Biopsieplanung verbessert sein.

Gemäß einem Aspekt der Erfindung weist der zweite Schichtbilddatensatz und der dritte Schichtbilddatensatz ausschließlich die Schwächungsinformation der mindestens einen Mikrokalzifikation auf. Die orthogonalen Projektionen aus den Tomosynthesedaten in Form des zweiten und dritten Schichtbilddatensatzes weisen insbesondere im Wesentlichen nur die detektierten Mikrokalzifikationen ohne den Brusthintergrund auf. Da das Ziel darin besteht, Mikrokalkcluster zu detektieren, können in einem Ausführungsbeispiel aufgrund der einfacheren Detektion bzw. Erkennbarkeit der zweite und der dritte Schichtbilddatensatz im Wesentlichen nur mit Mikrokalzifikationen befüllt sein und der gesamte Brusthintergrund nicht dargestellt werden. Der zweite und der dritte Schichtbilddatensatz können insbesondere im Wesentlichen nur mit den detektierten Mikrokalzifikationen befüllt werden. Vorteilhaft kann die Lage von mehreren Mikrokalzifikationen zueinander besonders einfach in einer übersichtlichen Darstellung bestimmt bzw. lokalisiert werden.

Gemäß einem Aspekt der Erfindung weist der zweite Schichtbilddatensatz und der dritte Schichtbilddatensatz ausschließlich die Schwächungsinformation des Drüsengewebes bzw. des Weichteilgewebes, oder die Schwächungsinformation des Drüsengewebes und der mindestens eine Kalzifikation auf. Die orthogonalen Projektionen der Tomosyntheseaufnahme in Form des zweiten und dritten Schichtbilddatensatzes weisen im Wesentlichen nur Drüsengewebe, insbesondere sogenannte Massen, und ggf. Mikrokalzifikationen auf. Vorteilhaft kann potentielles Tumorgewebe in den zweiten und dritten Schichtbildern angezeigt werden. Insbesondere die Kombination von Mikrokalzifikationen und Massen in einer Ansicht kann vorteilhaft für die Diagnose genutzt werden. Insbesondere bei wenigen detektierten Mikrokalzifikationen in der Mehrzahl von ersten Schichtbildern oder in dem ausgewählten ersten Schichtbilddatensatz können der zweite und der dritte Schichtbilddatensatz die Schwächungsinformation des Weichteilgewebes bzw. des Brusthintergrunds aufweisen. In einem Ausführungsbeispiel, im Besonderen für die Biopsie von Läsionen ohne vorhandene Mikrokalzifikationen oder mit sehr wenigen Mikrokalzifikationen, kann (auch) das Weichteilgewebe in den entsprechenden Schichtbildern dargestellt werden.

In einer Ausführungsform können alternativ oder zusätzlich zu den detektierten Mikrokalzifikationen im Wesentlichen alle in der Brust darstellbaren Strukturen von dem zweiten und dritten Schichtbilddatensatz umfasst sein. In einer Ausführungsform kann alternativ oder zusätzlich im Wesentlichen nur das Drüsengewebe, insbesondere inklusive potentieller Tumore, von dem zweiten und dritten Schichtbilddatensatz umfasst sein.

Gemäß einem Aspekt der Erfindung weist der erste Schichtbilddatensatz die gesamte Schwächungsinformation auf. Der ausgewählte erste Schichtbilddatensatz umfasst im Wesentlichen alle darstellbaren Schwächungsinformationen des Untersuchungsobjekts für diese Schicht, d.h. es können sowohl die detektierten Mikrokalzifikationen als auch das Weichteilgewebe dargestellt werden. Vorteilhaft kann der ausgewählte erste Schichtbilddatensatz ein Gesamtbild der Schwächungsinformationen des Objekts umfassen. Vorteilhaft kann bei der Diagnose sowohl die Information über Massen als auch Mikrokalzifikationen auf einen Blick aus dem ausgewählten ersten Schichtbilddatensatz gewonnen werden. Die Details über die Erstreckung von Läsionen bzw. Massen und Mikrokalzifikationsclustern kann insbesondere durch den zweiten und den dritten Schichtbilddatensatz bereitgestellt werden.

Gemäß einem Aspekt der Erfindung wird die markierte Mikrokalzifikation im ersten, zweiten und dritten Schichtbilddatensatz hervorgehoben. Eine ausgewählte Mikrokalzifikation oder eine interessierende Region kann im ersten, zweiten und/oder dritten Schichtbilddatensatz hervorgehoben werden, beispielsweise mittels einer farbigen Markierung. Vorteilhaft kann die Mikrokalzifikation und deren Lokalisation in verschiedenen Ansichten betrachtet werden.

Gemäß einem Aspekt der Erfindung weist der zweite und dritte Schichtbilddatensatz eine maximale Schichtdicke von im Wesentlichen 4cm auf. Mittels einer Tastenkombination und einen Klick auf die markierte Mikrokalzifikation oder einer anderen vorbestimmten Benutzereingabe hinsichtlich der markierten Mikrokalzifikation basierend auf dem ausgwählten ersten Schichtbilddatensatz kann im zweiten und dritten Schichtbilddatensatz nur ein engerer Bereich der Projektion dargestellt werden, beispielsweise +/- 2cm. Alternativ kann der engere Bereich zwischen +/- 1cm und +/-3cm liegen. Vorteilhaft können davor- oder dahinterliegende benachbarte Mikrokalzifikationen ausgeblendet werden. Dies kann insbesondere den Vorteil haben, dass bei einer Brust mit einer großen Anzahl von Mikrokalzifikationen diese sich im relevanten Bereich nicht überlagern.

Gemäß einem Aspekt der Erfindung wird eine Biopsieregion in dem ersten, zweiten oder/und dritten Schichtbilddatensatz markiert. Die Biopsieregion kann ein Cluster enthalten. Die Biopsieregion kann um das Cluster herum eine zusätzliche Hülle aufweisen, welche einen vordefinierten Abstand zum Cluster einhält, so dass das Cluster möglichst zuverlässig biopsiert werden kann. Die Biopsieregion kann manuell oder automatisch, beispielsweise basierend auf der Clusteranalyse, durchgeführt werden. Es kann eine Geweberegion, welche biopsiert werden soll, d.h. ein Biopsieregion, ausgewählt und markiert werden. Es kann eine Biopsieregion statt eines Zielpunkts für die Biopsie ausgewählt und markiert werden. Die Biopsieregion kann insbesondere in zwei oder in drei Ebenen auswählbar und markierbar sein. Beispielsweise kann die Biopsieregion im ausgewählten ersten Schichtbilddatensatz und im zweiten und/oder dritten Schichtbilddatensatz ausgewählt und markiert werden. Vorteilhaft kann bei der Markierung der Biopsieregion die verbesserte Lokalisation der Mikrokalzifikationen berücksichtigt werden.

Gemäß einem Aspekt der Erfindung wird eine virtuelle Überlagerung eines Modells einer ausgewählten Biopsienadel mit einer Entnahmeöffnung, einer sogenannten Notch, zumindest in dem ersten Schichtbilddatensatz in der markierten Biopsieregion angezeigt. Das Modell der Biospienadel kann insbesondere als dreidimensionales Modell für die Überlagerung zur Verfügung gestellt werden, so dass die Biopisenadel im ausgewählten ersten, im zweiten und im dritten Schichtbilddatensatz entsprechend angezeigt werden kann. Es können verschiedene verfügbare Modelle von Biopsienadeln, insbesondere mit einer sogenannten Notch, zur virtuellen Überlagerung auswählbar sein. Die ausgewählte Biopsienadel weist eine Entnahmeöffnung, die sogenannte Notch, auf. Die Entnahmeöffnung kann als seitliches Vakuum-Entnahmefenster an der Biopisenadel ausgebildet sein. Die Biopsie kann mittels Drehen das Biopsiematerial bzw. das suspekte Gewebe einsaugen. Vorteilhaft können alle benutzbaren Nadeltypen und Notches als 3D-Modelle in der Software abrufbar sein. Diese 3D-Modelle können auf einen vorhandenen Bilddatensatz projiziert bzw. virtuell überlagert dargestellt werden. Für einen Biopsie-Workflow kann zusätzlich zu den vorhandenen Schichtbilddaten, insbesondere in den ausgewählten ersten, zweiten und dritten Schichtbilddaten, eine virtuelle Überlagerung der ausgewählten Nadel und Notch in den Schichtbilddaten, insbesondere in den ausgewählten ersten, zweiten und dritten Schichtbilddaten, in der angewählten Zielposition bzw. Biopsieregion eingeblendet werden. Hierbei ist die Lage, Ausrichtung und das Größenverhältnis von dem zu biopsierendem Gewebe und der Notch von besonderer Bedeutung, d.h. die virtuelle Überlagerung kann insbesondere ein originalgetreues Größenverhältnis der Schichtbilddaten und der Nadel bzw. Notch aufweisen. Dies erlaubt dem Benutzer zu ermitteln, ob die richtige Nadelstärke, Notchgröße und der richtige bzw. optimale Zugangsweg bzw. Pfad der Nadel durch das umliegende Gewebe gewählt bzw. vorgeschlagen werden. Eine Korrektur von Biopsienadel, Nadelstärke, Notchgröße und Pfad wäre zu diesem Zeitpunkt vorteilhaft möglich, da die reale Biopsienadel noch nicht ins Gewebe eingeführt ist. Vorteilhaft können der zweite und der dritte Schichtbilddatensatz eine Biopsieplanung erleichtern.

Gemäß einem Aspekt der Erfindung wird eine Meldung ausgegeben, falls die ausgewählte Biopsienadel die markierte Biopsieregion unvollständig erfasst, oder/und für die markierte Biopsieregion ein Vorschlag einer geeigneten Biopsienadel oder/und eines Biopsiewinkels ausgegeben wird. Ein Algorithmus zur Planung der Biopsie kann auf Basis der Position und Ausdehnung des suspekten Bereichs bzw. der Biopsieregion eine Empfehlung für Biopsienadel und Notch sowie dem optimalen Zugang bzw. Pfad oder Winkel berechnen, dies kann anschließend auf der Anzeige visualisiert werden. Vorteilhaft kann die Biopsieplanung verbessert werden. Vorteilhaft kann eine Meldung oder ein ein Hinweis ausgegeben werden, falls der Benutzer eine aus Algorithmus-Sicht ungeeignete Biopsienadel, ungeeignete Notch oder einen ungeeigneten Zugangsweg verwenden will bzw. ausgewählt hat.

Gemäß einem Aspekt der Erfindung wird eine Trajektorie zur automatischen Biopsiesteuerung der Biopsienadel berechnet. Es kann eine Trajektorie bzw. ein Pfad zur Biopsiesteuerung basierend auf der markierten Biospieregion berechnet werden.

Insbesondere kann ein optimaler Biospiewinkel bestimmt werden. Die Berechnung des Biopsiewinkels kann neben dem optimalen Winkel zur Positionierung der Notch am bzw. in der Biopsieregion einen geeigneten Eintrittspfad bzw. eine geeignete Eintrittsstelle in die Brust umfassen. Auf Basis der Berechnung kann das Biopsiesystem nachfolgend automatisch gesteuert werden, so dass eine Biopsieeinheit mit der Biopsienadel an die korrekte Position verfahren wird, ggf. nach Bestätigung durch den Benutzer. Es kann die Trajektorie mittels Steuersignalen basierend auf der Berechnung gesteuert werden. Vorteilhaft kann die Biopsie genauer durchgeführt werden. Bei der Berechnung bzw. zu deren Visualisierung können insbesondere der zweite und der dritte Schichtbilddatensatz entsprechend des optimalen Winkels gegenüber der x- und y-Achse ermittelt werden. Der Schichtbilddatensatz kann dann entlang einer Achse, welche um den Winkel gegenüber der x- bzw. y-Achse gekippt ist, ermittelt werden. Vorteilhaft kann der optimale Winkel für die Trajektorie mit Hilfe der Ermittlung unterschiedlicher zweiter und dritter Schichtbilddatensätze mit unterschiedlichen Winkeln bestimmt werden.

Die Erfindung betrifft ferner ein Mammographiesystem aufweisend Mittel zum Durchführen eines erfindungsgemäßen Verfahrens. Das Mammographiesystem kann insbesondere eine Biopsieeinheit aufweisen. Das Mammographiesystem weist eine Aufnahmeeinheit zum Aufnehmen der Tomosyntheseaufnahme. Die Aufnahmeeinheit umfasst insbesondere die Röntgenquelle und den Röntgendetektor. Das Mammographiesystem weist eine Rekonstruktionseinheit zum Rekonstruieren eines Volumenbilds bzw. von einer Mehrzahl von ersten Schichtbildern auf. Die Rekonstruktionseinheit kann von der Recheneinheit umfasst sein. Das Mammographiesystem weist ferner eine Auswahleinheit zum Auswählen eines ersten Schichtbilddatensatzes aus der Mehrzahl von ersten Schichtbilddatensätzen auf. Das Mammographiesystem weist ferner eine Markierungseinheit zum Markieren einer Mikrokalzifikation oder einer interessierenden Region mit einer punktförmigen Markierung im ausgewählten ersten Schichtbilddatensatz auf. Die Auswahleinheit und die Markierungseinheit können eine, ggf. gemeinsame, Eingabeeinheit umfassen. Das Mammographiesystem weist ferner eine Ermittlungseinheit zum Ermitteln von einem zweiten Schichtbilddatensatz in einer zur ersten Ebene orthogonalen zweiten Ebene und von einem dritten Schichtbilddatensatz in einer zur ersten Ebene und zweiten Ebene orthogonalen dritten Ebene auf, wobei der Schnittpunkt der ersten Ebene, der zweiten Ebene und der dritten Ebene die punktförmige Markierung umfasst. Die Vorteile des erfindungsgemäßen Verfahrens können vorteilhaft auf das Mammographiesystem übertragen werden. Das Mammographiesystem kann bevorzugt eine lokale oder entfernte Anzeigeeinheit zur Darstellung des ausgewählten ersten, zweiten und dritten Schichtbilddatensatzes aufweisen.

Die Erfindung betrifft ferner ein Computerprogrammprodukt mit einem Computerprogramm, welches direkt in eine Speichereinrichtung einer Steuereinrichtung eines Mammographiesystems ladbar ist, mit Programmabschnitten, um alle Schritte eines erfindungsgemäßen Verfahrens auszuführen, wenn das Computerprogramm in der Steuereinrichtung des Mammographiesystems ausgeführt wird.

Die Erfindung betrifft ferner ein computerlesbares Medium, auf welchem von einer Rechnereinheit einlesbare und ausführbare Programmabschnitte gespeichert sind, um alle Schritte eines erfindungsgemäßen Verfahrens auszuführen, wenn die Programmabschnitte von dem Mammographiesystem ausgeführt werden.

Nachfolgend werden Ausführungsbeispiele der Erfindung anhand von Zeichnungen näher erläutert. Hierbei zeigt:
FIG 1 eine schematische Darstellung des erfindungsgemäßen Verfahrens;
FIG 2 eine schematische Darstellung der erfindungsgemäßen Darstellung auf einer Anzeigeeinheit eines Mammographiesytems;
FIG 3 eine schematische Darstellung einer virtuellen Überlagerung einer Biopsienadel in einem ersten Schichtbilddatensatz in einer ersten Ausführungsform;
FIG 4 eine schematische Darstellung einer virtuellen Überlagerung einer Biopsienadel in einem ersten Schichtbilddatensatz in einer zweiten Ausführungsform; und
FIG 5 eine schematische Darstellung eines erfindungsgemäßen Mammographiesystems.

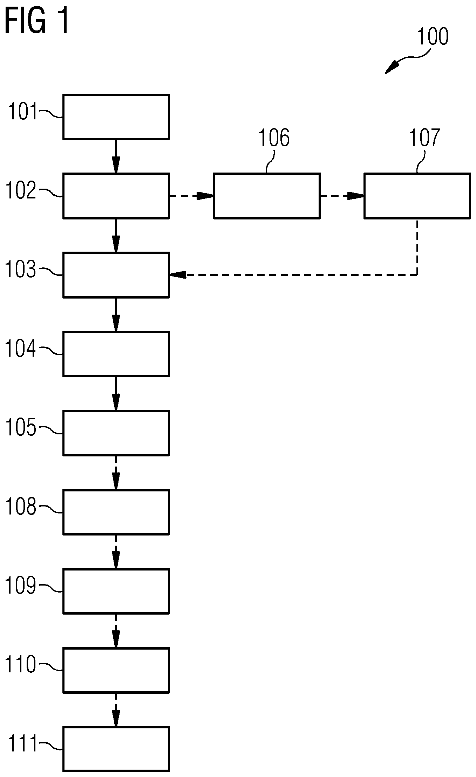

Die Fig. 1 zeigt eine beispielhafte Ausführung des erfindungsgemäßen Verfahrens 100. Das Verfahren 100 zur Ermittlung orthogonaler Schichtbilddatensätze einer Tomosyntheseaufnahme eines Untersuchungsbereichs, insbesondere einer Brust, weist die Schritte des Aufnehmens 101, des Rekonstruierens 102, des Auswählens 103, des Markierens 104 und des Ermittelns 105 auf. Im Schritt des Aufnehmens 101 wird eine Tomosyntheseaufnahme aufgenommen. Im Schritt des Rekonstruierens 102 wird eine Mehrzahl von ersten Schichtbilddatensätzen in einer ersten Ebene basierend auf der Tomosyntheseaufnahme rekonstruiert. Im Schritt des Auswählens 103 wird ein erster Schichtbilddatensatzes aus der Mehrzahl von ersten Schichtbilddatensätzen ausgewählt. Im Schritt des Markierens 104 wird ein Mikrokalzifikation oder eine interessierende Region mit einer punktförmigen Markierung im ausgewählten ersten Schichtbilddatensatz markiert. Im Schritt des Ermittelns 105 wird ein zweiter Schichtbilddatensatz in einer zur ersten Ebene orthogonalen zweiten Ebene und ein dritter Schichtbilddatensatz in einer zur ersten Ebene und zweiten Ebene orthogonalen dritten Ebene ermittelt, wobei der Schnittpunkt der ersten Ebene, der zweiten Ebene und der dritten Ebene die punktförmige Markierung umfasst. Das Verfahren kann ferner einen Schritt des Anzeigens umfassen, insbesondere von dem ausgewählten ersten, zweiten und dritten Schichtbilddatensatz.

Die mindestens eine Mikrokalzifikation kann ferner in einem Schritt des Detektierens 106 automatisch oder ggf. manuell in den ersten Schichtbilddatensätzen detektiert werden. In einem Schritt der Clusteranalyse 107 kann bei einer Mehrzahl detektierter Mikrokalizifikationen eine automatische Clusteranalyse 107 durchgeführt werden.

Der zweite Schichtbilddatensatz und der dritte Schichtbilddatensatz können ausschließlich die Schwächungsinformation der mindestens einen Mikrokalzifikation aufweisen. Alternativ können der zweite Schichtbilddatensatz und der dritte Schichtbilddatensatz ausschließlich die Schwächungsinformation des Drüsengewebes aufweisen. Alternativ können der zweite Schichtbilddatensatz und der dritte Schichtbilddatensatz die Schwächungsinformation des Drüsengewebes und der mindestens einen Mikrokalzifikation aufweisen. Der erste Schichtbilddatensatz weist bevorzugt die gesamte Schwächungsinformation auf. Die markierte Mikrokalzifikation wird bevorzugt im ersten, zweiten und dritten Schichtbilddatensatz hervorgehoben. In einer Ausführungsform können der zweite und dritte Schichtbilddatensatz eine maximale Schichtdicke von 4cm aufweisen.

In einem optionalen Schritt des Markierens 108 einer Biopsieregion kann eine Biopsieregion in dem ersten, zweiten oder/und dritten Schichtbilddatensatz markiert werden. In einem optionalen Schritt der virtuellen Überlagerung 109 kann ein Modell einer ausgewählten Biopsienadel mit einer Entnahmeöffnung zumindest in dem ersten Schichtbilddatensatz in der markierten Biopsieregion angezeigt werden. In einem optionalen Schritt der Überprüfung 110 kann eine Meldung ausgegeben werden, falls die ausgewählte Biopsienadel die markierte Biopsieregion unvollständig erfasst, oder/und für die markierte Biopsieregion ein Vorschlag einer geeigneten Biopsienadel oder/und eines Biopsiewinkels ausgegeben werden. In einem optionalen Schritt der Berechnung 111 kann eine Trajektorie zur automatischen Biopsiesteuerung der Biopsienadel berechnet werden.

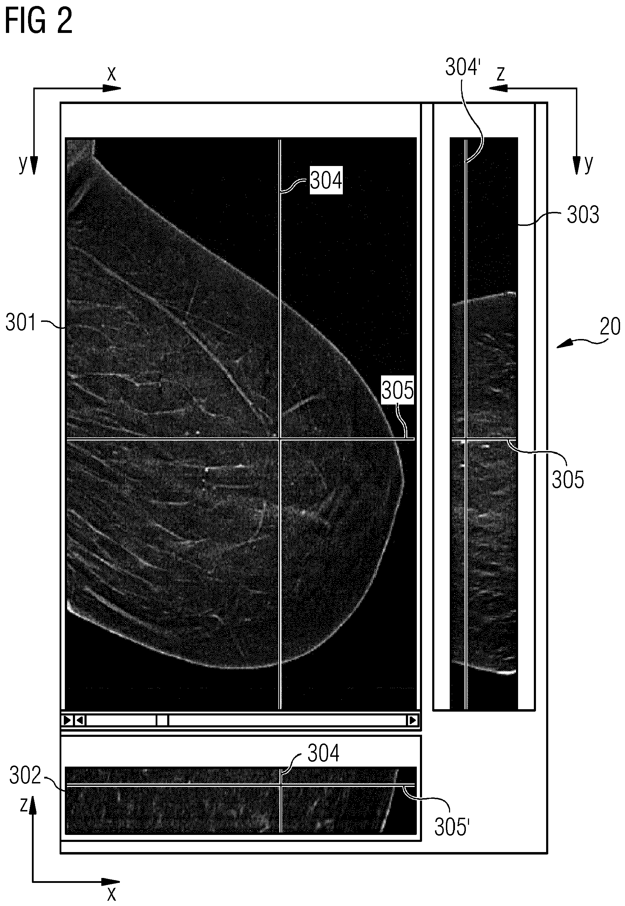

Die Fig. 2 zeigt eine beispielshafte Ausführung der erfindungsgemäßen Darstellung auf einer Anzeigeeinheit eines Mammographiesytems. Die Anzeigeeinheit kann die Ausgabeeinheit 20 sein. In einem Hauptbereich wird der ausgewählte erste Schichtbilddatensatz 301 in einer xy-Ebene angezeigt. Eine erste vertikale Markierungslinie 304 und eine zweite horizontale Markierungslinie 305 schneiden sich, an dieser Schnittstelle liegt die punktförmige Markierung. In einem Nebenbereich, beispielsweise darunter mit gleicher x-Skala angeordnet, wird der zweite Schichtbilddatensatz 302 in einer xz-Ebene angezeigt. Die erste Markierungsline 304 ist an der gleichen x-Position wie im ausgewählten ersten Schichtbilddatensatz 301 angezeigt. Die zweite Markierungsline 305' ist an der z-Position des ausgewählten ersten Schichtbilddatensatzes 301 angezeigt. In einem Nebenbereich, beispielsweise neben dem Hauptbereich mit gleicher y-Skala angeordnet, wird der dritte Schichtbilddatensatz 303 in einer yz-Ebene angezeigt. Die zweite Markierungsline 305 ist an der gleichen y-Position wie im ausgewählten ersten Schichtbilddatensatz 301 angezeigt. Die erste Markierungsline 304' ist an der z-Position des ausgewählten ersten Schichtbilddatensatzes 301 angezeigt. Der ausgewählte erste Schichtbilddatensatz 301, zweite Schichtbilddatensatz 302 und dritte Schichtbilddatensatz 303 zeigen sowohl Mikrokalzifikationen, welche als weiße Punkte erkennbar sind, sowie das Weichteilgewebe bzw. den Brusthintergrund.

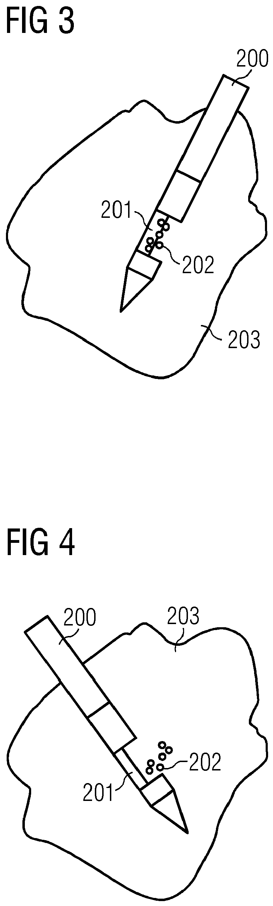

Die Fig. 3 zeigt eine beispielshafte Ausführung einer virtuellen Überlagerung einer Biopsienadel 200 in einem ersten Schichtbilddatensatz in einer ersten Ausführungsform. Sowohl die Größe der Biopisenadel 200, die Größe der Notch 201 und auch der Pfad bzw. die Trajektorie sind auf die Biopiseregion mit den Mikrokalzifkationen 202 in einem Gewebe 203 optimal abgestimmt.

Die Fig. 4 zeigt eine beispielshafte Ausführung einer virtuellen Überlagerung einer Biopsienadel 200 in einem ersten Schichtbilddatensatz in einer zweiten Ausführungsform. Der Pfad bzw. die Trajektorie oder der Zugangsweg sind nicht ideal. Die Nadelgröße der Biopsienadel 200 und die Größe der Notch 201 sind nicht ausreichend für die Extraktion des vollständigen Clusters aufweisend die Mirkokalzifikationen 202.

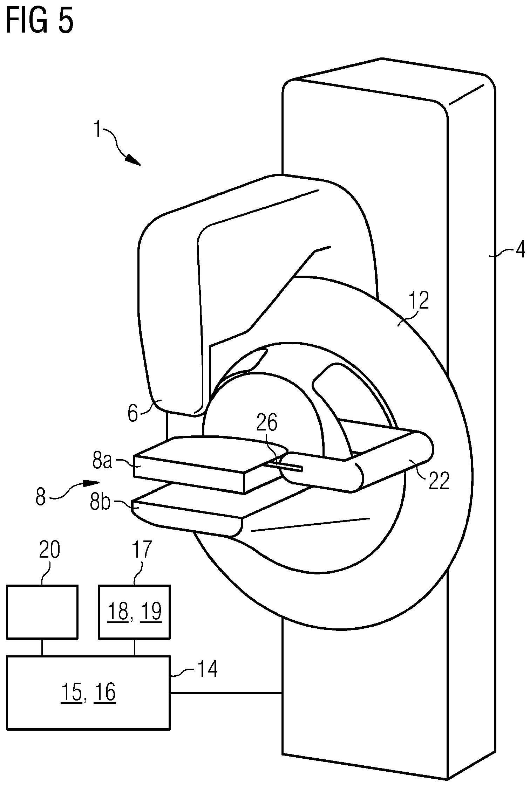

Die Fig. 5 zeigt eine beispielshafte Ausführung eines erfindungsgemäßen Mammographiesystems 1. Das Mammographiesystem 1 umfasst eine Standeinheit 4 mit einem in einem vorbestimmten Winkelbereich rotierbaren Röntgenarm und einer daran angeordneten Röntgenquelle 6. An der Standeinheit 4 ist ferner eine Rotationseinheit 12 angeordnet, an welcher sowohl die Kompressionseinheit 8 als auch die Biopsieeinheit 22 rotierbar zum Einstellen einer Kompressionsrichtung bzw. eines Biopsiepfads angeordnet sind. Die Kompressionseinheit 8 umfasst das Kompressionselement 8a und den Röntgendetektor 8b, zwischen denen die Brust zur Aufnahme positioniert und komprimiert wird. An der Biopsieeinheit 22 ist eine Biopsienadel 26 angeordnet. Das Mammographiesystem umfasst eine Recheneinheit 14 mit einer Rekonstruktionseinheit 15 und einer Ermittlungseinheit 16. Mit der Recheneinheit ist eine Eingabeeinheit 17 verbunden, welche die Auswahleinheit 18 und die Markierungseinheit 19 umfasst. Die Eingabeeinheit 17 kann insbesondere eine Benutzerschnittstelle, beispielsweise ein Maus oder ein Touch User Interface umfassen. Mit der Recheneinheit 14 ist ferner die Ausgabeeinheit 20 verbunden, welche eine Anzeigeeinheit bzw. einen Bildschirm umfassen kann.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren (100) zur Ermittlung orthogonaler Schichtbilddatensätze einer Tomosyntheseaufnahme eines Untersuchungsbereichs, insbesondere einer Brust, aufweisend die Schritte:
- Aufnehmen (101) einer Tomosyntheseaufnahme und Rekonstruieren (102) von einer Mehrzahl von ersten Schichtbilddatensätzen in einer ersten Ebene basierend auf der Tomosyntheseaufnahme,
- Auswählen (103) eines ersten Schichtbilddatensatzes (301) aus der Mehrzahl von ersten Schichtbilddatensätzen,
- Markieren (104) einer Mikrokalzifikation (202) oder einer interessierenden Region mit einer punktförmigen Markierung im ausgewählten ersten Schichtbilddatensatz,
- Ermitteln (105) von einem zweiten Schichtbilddatensatz (302) in einer zur ersten Ebene orthogonalen zweiten Ebene und von einem dritten Schichtbilddatensatz (303) in einer zur ersten Ebene und zweiten Ebene orthogonalen dritten Ebene, wobei der Schnittpunkt der ersten Ebene, der zweiten Ebene und der dritten Ebene die punktförmige Markierung umfasst.

2. Verfahren nach einem der vorangehenden Ansprüche, wobei mindestens eine Mikrokalzifikation (202) automatisch detektiert wird.

3. Verfahren nach einem der vorangehenden Ansprüche, wobei bei einer Mehrzahl detektierter Mikrokalizifikationen eine automatische Clusteranalyse durchgeführt wird.

4. Verfahren nach einem der vorangehenden Ansprüche, wobei der zweite Schichtbilddatensatz und der dritte Schichtbilddatensatz ausschließlich die Schwächungsinformation der mindestens einen Mikrokalzifikation aufweist.

5. Verfahren nach einem der Ansprüche 1 bis 3, wobei der zweite Schichtbilddatensatz und der dritte Schichtbilddatensatz ausschließlich die Schwächungsinformation des Drüsengewebes, oder die Schwächungsinformation des Drüsengewebes und der mindestens einen Mikrokalzifikation aufweist.

6. Verfahren nach einem der vorangehenden Ansprüche, wobei der erste Schichtbilddatensatz die gesamte Schwächungsinformation aufweist.

7. Verfahren nach einem der vorangehenden Ansprüche, wobei die markierte Mikrokalzifikation im ersten, zweiten und dritten Schichtbilddatensatz hervorgehoben wird.

8. Verfahren nach einem der vorangehenden Ansprüche, wobei der zweite Schichtbilddatensatz und der dritte Schichtbilddatensatz eine maximale Schichtdicke von 4cm aufweist.

9. Verfahren nach einem der vorangehenden Ansprüche, wobei eine Biopsieregion in dem ersten Schichtbilddatensatz, dem zweiten Schichtbilddatensatz oder/und dem dritten Schichtbilddatensatz markiert wird.

10. Verfahren nach Anspruch 9, wobei eine virtuelle Überlagerung eines Modells einer ausgewählten Biopsienadel (200) mit einer Entnahmeöffnung zumindest in dem ersten Schichtbilddatensatz in der markierten Biopsieregion angezeigt wird.

11. Verfahren nach Anspruch 9 oder 10, wobei eine Meldung ausgegeben wird, falls die ausgewählte Biopsienadel die markierte Biopsieregion unvollständig erfasst, oder/und für die markierte Biopsieregion ein Vorschlag einer geeigneten Biopsienadel oder/und eines Biopsiewinkels ausgegeben wird.

12. Verfahren nach einem der Ansprüche 9 bis 11, wobei eine Trajektorie zur automatischen Biopsiesteuerung der Biopsienadel berechnet wird.

13. Mammographiesystem (1) aufweisend Mittel zum Durchführen eines Verfahrens nach einem der Ansprüche 1 bis 12.

14. Computerprogrammprodukt mit einem Computerprogramm, welches direkt in eine Speichereinrichtung einer Steuereinrichtung eines Mammographiesystems ladbar ist, mit Programmabschnitten, um alle Schritte eines Verfahrens nach einem der Ansprüche 1 bis 12 auszuführen, wenn das Computerprogramm in der Steuereinrichtung des Mammographiesystems ausgeführt wird.

15. Computerlesbares Medium, auf welchem von einer Rechnereinheit einlesbare und ausführbare Programmabschnitte gespeichert sind, um alle Schritte eines Verfahrens nach einem der Ansprüche 1 bis 12 auszuführen, wenn die Programmabschnitte von dem Mammographiesystem ausgeführt werden.
